# EUROPEAN PATENT APPLICATION

(11) **EP 2 774 986 A1**
(43) Date of publication of application: **10.09.2014**
(21) Application number: 13158012.8
(22) Date of filing: 06.03.2013
(51) Int. Cl.: C12N 9/04, C12N 9/88, C12P 7/16

(54) **Improvement of clostridial butanol production by gene overexpression**

(71) Applicant: Clariant Produkte (Deutschland) GmbH, 65929 Frankfurt am Main (DE); TU München, 80333 München (DE)
(72) Inventor: Dragovic, Zdravko, 81375 München (DE); Schirrmacher, Georg, 80538 München (DE); Lütke-Eversloh, Tina, 18057 Rostock (DE); Mann. Miriam, 18057 Rostock (DE); Scmidt, Michael, 85748 Garching (DE); Weuster-Botz, Dirk, 80634 München (DE)
(74) Representative: Silber, Anton

(57) **Abstract**

The present invention relates to metabolic engineering of *Clostridium acetobutylicum.* A Clostridium cell with the ability to produce butanol, wherein the cell comprises a genetic modification that results in overexpression of one or more of the following genes:
● a gene encoding crotonase (*crt*),
● a gene encoding butyryl-CoA dehydrogenase (*bcd*),
● a gene encoding 3-hydroxybutyryl-CoA dehydrogenase (*hbd*).

Overexpression of any of the two enzymes increased the speed of butanol production for at least factor of two, resulting in increase of final butanol titer for at least 28%.

SEQUENCE LISTING

<110>  Clariant Produkte (Deutschland) GMBH

<120>  Improvement of clostridial butanol production by gene
       overexpression

<130>  163300

<160>  4

<170>  PatentIn version 3.5

<210>  1
<211>  261
<212>  PRT
<213>  Clostridium acetobutylicum

<400>  1

Met Glu Leu Asn Asn Val Ile Leu Glu Lys Glu Gly Lys Val Ala Val
1               5                   10                  15

Val Thr Ile Asn Arg Pro Lys Ala Leu Asn Ala Leu Asn Ser Asp Thr
            20                  25                  30

Leu Lys Glu Met Asp Tyr Val Ile Gly Glu Ile Glu Asn Asp Ser Glu
        35                  40                  45

Val Leu Ala Val Ile Leu Thr Gly Ala Gly Glu Lys Ser Phe Val Ala
    50                  55                  60

Gly Ala Asp Ile Ser Glu Met Lys Glu Met Asn Thr Ile Glu Gly Arg
65                  70                  75                  80

Lys Phe Gly Ile Leu Gly Asn Lys Val Phe Arg Arg Leu Glu Leu Leu
                85                  90                  95

Glu Lys Pro Val Ile Ala Ala Val Asn Gly Phe Ala Leu Gly Gly Gly
            100                 105                 110

Cys Glu Ile Ala Met Ser Cys Asp Ile Arg Ile Ala Ser Ser Asn Ala
        115                 120                 125

Arg Phe Gly Gln Pro Glu Val Gly Leu Gly Ile Thr Pro Gly Phe Gly
    130                 135                 140

Gly Thr Gln Arg Leu Ser Arg Leu Val Gly Met Gly Met Ala Lys Gln
145                 150                 155                 160

Leu Ile Phe Thr Ala Gln Asn Ile Lys Ala Asp Glu Ala Leu Arg Ile
                165                 170                 175

Gly Leu Val Asn Lys Val Val Glu Pro Ser Glu Leu Met Asn Thr Ala
        180                 185                 190

Lys Glu Ile Ala Asn Lys Ile Val Ser Asn Ala Pro Val Ala Val Lys
    195                 200                 205

Leu Ser Lys Gln Ala Ile Asn Arg Gly Met Gln Cys Asp Ile Asp Thr
    210                 215                 220

Ala Leu Ala Phe Glu Ser Glu Ala Phe Gly Glu Cys Phe Ser Thr Glu
225                 230                 235                 240

Asp Gln Lys Asp Ala Met Thr Ala Phe Ile Glu Lys Arg Lys Ile Glu
                245                 250                 255

Gly Phe Lys Asn Arg
        260

<210>  2
<211>  379
<212>  PRT
<213>  Clostridium acetobutylicum

<400>  2

Met Asp Phe Asn Leu Thr Arg Glu Gln Glu Leu Val Arg Gln Met Val
1               5                   10                  15

Arg Glu Phe Ala Glu Asn Glu Val Lys Pro Ile Ala Ala Glu Ile Asp
        20                  25                  30

Glu Thr Glu Arg Phe Pro Met Glu Asn Val Lys Lys Met Gly Gln Tyr
        35                  40                  45

Gly Met Met Gly Ile Pro Phe Ser Lys Glu Tyr Gly Gly Ala Gly Gly
    50                  55                  60

Asp Val Leu Ser Tyr Ile Ile Ala Val Glu Glu Leu Ser Lys Val Cys
65                  70                  75                  80

Gly Thr Thr Gly Val Ile Leu Ser Ala His Thr Ser Leu Cys Ala Ser
                85                  90                  95

Leu Ile Asn Glu His Gly Thr Glu Glu Gln Lys Gln Lys Tyr Leu Val
        100                 105                 110

Pro Leu Ala Lys Gly Glu Lys Ile Gly Ala Tyr Gly Leu Thr Glu Pro
    115                 120                 125

Asn Ala Gly Thr Asp Ser Gly Ala Gln Gln Thr Val Ala Val Leu Glu
    130                 135                 140

Gly Asp His Tyr Val Ile Asn Gly Ser Lys Ile Phe Ile Thr Asn Gly
145                 150                 155                 160

Gly Val Ala Asp Thr Phe Val Ile Phe Ala Met Thr Asp Arg Thr Lys
                165                 170                 175

Gly Thr Lys Gly Ile Ser Ala Phe Ile Ile Glu Lys Gly Phe Lys Gly
            180                 185                 190

Phe Ser Ile Gly Lys Val Glu Gln Lys Leu Gly Ile Arg Ala Ser Ser
        195                 200                 205

Thr Thr Glu Leu Val Phe Glu Asp Met Ile Val Pro Val Glu Asn Met
    210                 215                 220

Ile Gly Lys Glu Gly Lys Gly Phe Pro Ile Ala Met Lys Thr Leu Asp
225                 230                 235                 240

Gly Gly Arg Ile Gly Ile Ala Ala Gln Ala Leu Gly Ile Ala Glu Gly
                245                 250                 255

Ala Phe Asn Glu Ala Arg Ala Tyr Met Lys Glu Arg Lys Gln Phe Gly
            260                 265                 270

Arg Ser Leu Asp Lys Phe Gln Gly Leu Ala Trp Met Met Ala Asp Met
        275                 280                 285

Asp Val Ala Ile Glu Ser Ala Arg Tyr Leu Val Tyr Lys Ala Ala Tyr
    290                 295                 300

Leu Lys Gln Ala Gly Leu Pro Tyr Thr Val Asp Ala Ala Arg Ala Lys
305                 310                 315                 320

Leu His Ala Ala Asn Val Ala Met Asp Val Thr Thr Lys Ala Val Gln
                325                 330                 335

Leu Phe Gly Gly Tyr Gly Tyr Thr Lys Asp Tyr Pro Val Glu Arg Met
            340                 345                 350

Met Arg Asp Ala Lys Ile Thr Glu Ile Tyr Glu Gly Thr Ser Glu Val
        355                 360                 365

Gln Lys Leu Val Ile Ser Gly Lys Ile Phe Arg
    370                 375

<210>  3
<211>  282
<212>  PRT
<213>  Clostridium acetobutylicum

<400>  3

Met Lys Lys Val Cys Val Ile Gly Ala Gly Thr Met Gly Ser Gly Ile
1               5                   10                  15

Ala Gln Ala Phe Ala Ala Lys Gly Phe Glu Val Val Leu Arg Asp Ile
            20                  25                  30

Lys Asp Glu Phe Val Asp Arg Gly Leu Asp Phe Ile Asn Lys Asn Leu
        35                  40                  45

Ser Lys Leu Val Lys Lys Gly Lys Ile Glu Glu Ala Thr Lys Val Glu
    50                  55                  60

Ile Leu Thr Arg Ile Ser Gly Thr Val Asp Leu Asn Met Ala Ala Asp
65                  70                  75                  80

Cys Asp Leu Val Ile Glu Ala Ala Val Glu Arg Met Asp Ile Lys Lys
                85                  90                  95

Gln Ile Phe Ala Asp Leu Asp Asn Ile Cys Lys Pro Glu Thr Ile Leu
            100                 105                 110

Ala Ser Asn Thr Ser Ser Leu Ser Ile Thr Glu Val Ala Ser Ala Thr
        115                 120                 125

Lys Thr Asn Asp Lys Val Ile Gly Met His Phe Phe Asn Pro Ala Pro
    130                 135                 140

Val Met Lys Leu Val Glu Val Ile Arg Gly Ile Ala Thr Ser Gln Glu
145                 150                 155                 160

Thr Phe Asp Ala Val Lys Glu Thr Ser Ile Ala Ile Gly Lys Asp Pro
                165                 170                 175

Val Glu Val Ala Glu Ala Pro Gly Phe Val Val Asn Arg Ile Leu Ile
            180                 185                 190

Pro Met Ile Asn Glu Ala Val Gly Ile Leu Ala Glu Gly Ile Ala Ser
        195                 200                 205

Val Glu Asp Ile Asp Lys Ala Met Lys Leu Gly Ala Asn His Pro Met
    210                 215                 220

Gly Pro Leu Glu Leu Gly Asp Phe Ile Gly Leu Asp Ile Cys Leu Ala
225                 230                 235                 240

Ile Met Asp Val Leu Tyr Ser Glu Thr Gly Asp Ser Lys Tyr Arg Pro
                245                 250                 255

His Thr Leu Leu Lys Lys Tyr Val Arg Ala Gly Trp Leu Gly Arg Lys
            260                 265                 270

Ser Gly Lys Gly Phe Tyr Asp Tyr Ser Lys
        275                 280

<210>  4
<211>  135
<212>  DNA
<213>  Clostridium acetobutylicum

<400>  4
tcgacttttt aacaaaatat attgataaaa ataataatag tgggtataat taagttgtta      60

gagaaaacgt ataaattagg gataaactat ggaacttatg aaatagattg aaatggttta     120

tctgttaccc cgtag                                                      135

## Description

### FIELD OF THE INVENTION

The present invention is related to the field of industrial microbiology and genetic engineering of organisms. More specifically the present invention relates to metabolic engineering of Clostridia such as *Clostridium acetobutylicum.* Even more specifically, the invention relates to overexpression of enzymes from the butanol metabolic pathway, namely crotonase (Crt), butyryi-CoA dehydrogenase (Bcd) and/or 3-hydroxybutyryl-CoA dehydrogenase (Hbd).

### BACKGROUND OF THE INVENTION

Butanol is an advanced biofuel because it can be used in existing gasoline-powered vehicles and other liquid-fueled processes without technical modifications. Historically, the so-called acetone-butanol-ethanol (ABE) fermentation with strains from genus *Clostridium* was employed since 1920s for industrial butanol and acetone production. However, industrial ABE fermentation declined rapidly after the 1950s as a result of the cheaper petrochemical production routes. Given today's knowledge on economic and environmental issues of fossil oil resources and increasing environmental problems due to global warming, biotechnological routes for energy production become more and more important. Therefore, so-called solventogenic clostridia have regained much interest recently due to their unique metabolic capacity of n-butanol production from renewable resources.

The genus *Clostridium* represents a diverse group of strictly anaerobic Gram-positive bacteria with the ability to form heat-resistant endospores for long-term survival under unfavorable environmental conditions. Besides several toxin-producing species such as *Clostridium perfringens, C. botulinum* or C. *tetani,* a large number of terrestrial non-pathogenic species show biotechnologically interesting metabolic properties like the formation of ethanol, butanol, acetone, isopropanol or propanediol. Although many clostridial strains are capable of solvent production in various amounts and ratios, the major producers are *C. acetobutylicum, C. beijerinckii, C. saccharoperbutylacetonicum* and *C. saccharobutylicum,* with *C. acetobutylicum* being the model organism of solvent producing clostridia for scientific research (Dürre P. 2011. Fermentative production of butanol - the academic perspective. Curr Opin Biotechnol 22:331-336.)

The fermentation of sugars by clostridia typically causes three different growth phases: (i) exponential growth and formation of acids, (ii) transition to stationary growth phase with reassimilation of acids and concomitant formation of solvents and (iii) formation of endospores. Sugars such as glucose or xylose are catabolized to pyruvate, and acetyl-CoA is primarily formed by the pyruvate:ferredoxin oxidoreductase. Under certain growth conditions such as pH values > 5 and iron limitation, lactate can be the major fermentation product. Usually, *C. acetobutylicum* synthesizes acetate via phosphotransacetylase and acetate kinase reactions with the latter reaction providing ATP. For the biosynthesis of butyrate, two molecules of acetyl-CoA are condensed to acetoacetyl-CoA, followed by a reduction to butyryl-CoA, which is then converted to butyrate via phosphotransbutyrylase and butyrate kinase reactions with ATP generation. In the butyrate/butanol biosynthetic pathway, acetyl-CoA is first converted to butyryl-CoA via thiolase, 3-hydroxybutyryl-CoA dehydrogenase, crotonase and butyryl-CoA dehydrogenase reactions. Subsequently, butyryl-CoA is converted to butyrate in the acidogenic and to butanol in the solventogenic phase, the latter catalyzed by combined aldehyde/alcohol dehydrogenase activities.

Metabolic engineering efforts to improve butanol production have been of limited success (reviewed by Lütke-Eversloh, Bahl 2011, Metabolic engineering of Clostridium acetobutylicum: recent advances to improve butanol production, Curr Opin Biotechnol 22:634-647; and Papoutsakis ET; 2008, Engineering solventogenic clostridia; Curr Opin Biotechnol 19:420-429.) .)

The studies on overexpression of butanol biosynthesis pathway genes were to date mainly focused on heterologous overexpression in organisms other than Clostrida: *E.coli* (Atsumi S et al 2007, Metab Eng 10(6): 305-11), *Lactobacillus brevis* (RU2375451), Pseudomonas and Bacillus (Nielsen DR et al 2009 Metab Eng 11(4-5):262-73). However the maximum obtained butanol concentrations were far below the *wt Clostridium acetobutylicum* level.

Metabolic engineering of *Clostridium acetobutylicum* strain M5 was done by overexpression of thiolase (Thl) combined with overexpression of alcohol dehydrogenase (Adh). The modification of the strain resulted in less side products as acetone but the maximal achievable concentration of the butanol remained at the wildtype level (Sillers R, Chow A, Tracy B, Papoutsakis ET. 2008. Metabolic engineering of the non-sporulating, non-solventogenic Clostridium acetobutylicum strain M5 to produce butanol without acetone demonstrate the robustness of the acid-formation pathways and the importance of the electron balance. Metab Eng 10:321-332.). In a similar study, Adh and Thl overexpression coupled with CoA transferase downregeulation lead to higher alcohol titers, but improved butanol titers were not achieved (Sillers R et al, 2009, Biotechnol Bioeng 102:38-49).

WO2012045022 describes the overexpression of alcohol/aldehyde dehydrogenase and butanol dehydrogenase in a non-butanol producing strain of *Clostridum tyrobutyricum.* The production of butanol was demonstrated, however at a lower level than *wt Clostridium acetobutylicum.* A similar approach has been tried in application KR20110033086. Comparable data are shown in (Sillers R et al, 2008 Metab Eng 10:321-332.), where no significant increase in butanol production was achieved. A slightly different approach was described in CN10261979. In addition to Adh, overexpression tests with Acetoacetyl-acylCoA transferase were performed. However, the achieved increase of butanol level was not significant.

Another problem of prior metabolic engineering approaches to improve clostridial butanol production was a concomitant increase of the acetate level which is not useable as fuel and thus undesirable for industrial production.

As a consequence of the limited success of rational metabolic engineering approaches to improve clostridial butanol production, Lütke-Eversloh, Bahl 2011, Metabolic engineering of Clostridium acetobutylicum: recent advances to improve butanol production. Curr Opin Biotechnol 22, 634-647, recommend an alternative approach to provide improved Clostridia strains, namely by selecting improved strains because of their phenotypic characteristics.

### PROBLEM TO BE SOLVED

The object of the present invention is thus to provide an improved process for the microbial production of butanol, and to provide microbial cells for such a process.

### SUMMARY OF THE INVENTION

The inventors have surprisingly found that the overexpression of specific enzymes involved in clostridial butanol production not only results in high butanol titer and faster kinetics, but also fails to lead to a significant increase in the acetate level. The inventors could also successfully show the scale-up of this approach to reactor scale.

The invention thus provides a method for producing butanol, comprising the steps of fermenting a medium containing a sugar, or a precursor thereof, with a Clostridium cell with the ability to produce butanol, wherein the cell comprises a genetic modification that results in overexpression of one or more of the following genes:
- a gene encoding crotonase (crt),
- a gene encoding butyryl-CoA dehydrogenase (bcd),
- a gene encoding 3-hydroxybutyryl-CoA dehydrogenase (hbd).

The invention further provides a Clostridium cell or strain as defined above, a method for its production, including a vector for production of such cells.

### BRIEF DESCRIPTION OF THE FIGURES

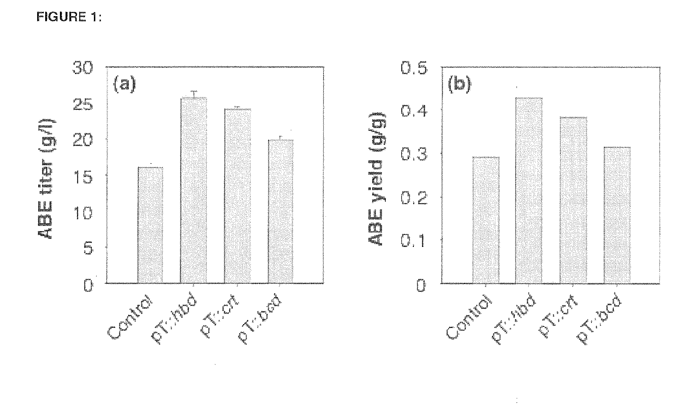

**Figure 1** shows product concentrations of (Acetone-Butanol-Ethanol = ABE) and, yields on glucose of recombinant *C*. *acetobutylicum-strains.* **Figure 1** **legend: Acetone-butanol-ethanol (ABE) titers (a) and yields on glucose (b) of recombinant** ***C.** acetobutylicum* **strains.** Cultivations were performed as described in Example 2, average data of three independent experiments are shown. Control, *C. acetobutylicum* ATCC 824/pT (vector control); *pT::hbd, C. acetobutylicum* ATCC 824/pT::hbd, pT::*crt,* C. *acetobutylicum* ATCC 824/pT::*crt* pT::*bcd, C*. *acetobutyticum* ATCC 824/pT::bcd.

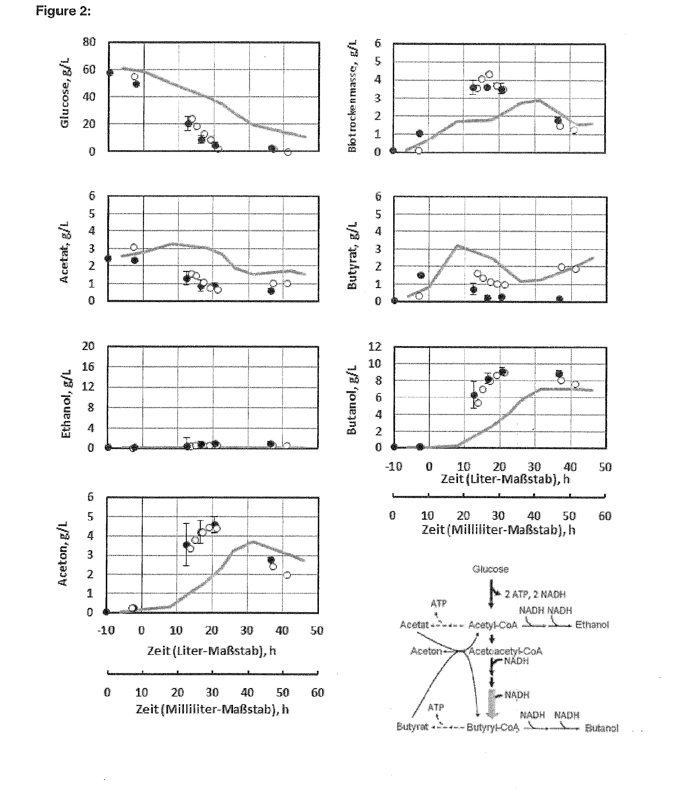

**Figure 2** shows changes in product concentrations during the batch cultivation in stirred tank reactors of *C*. *acetobutylicum:* OverExpression--pT::bcd are shown and compared to the wild type strains performance. **Figure 2** **legend: Characterization of** ***C.** acetobutylicum* **bcd overexpression strain:** Utilization of glucose, production of biomass, production of organic acids acetate, butyrate, and production of solvents acetone, ethanol and butanol is shown. White circles present performance of the bcd overexpression strain in liter scale reactor and black circles present strain performance in milliliter scale reactor. Performance of maternal strain with *wt* genotype is presented with grey line. Error bars stay for standard deviation from 3 independent experiments.

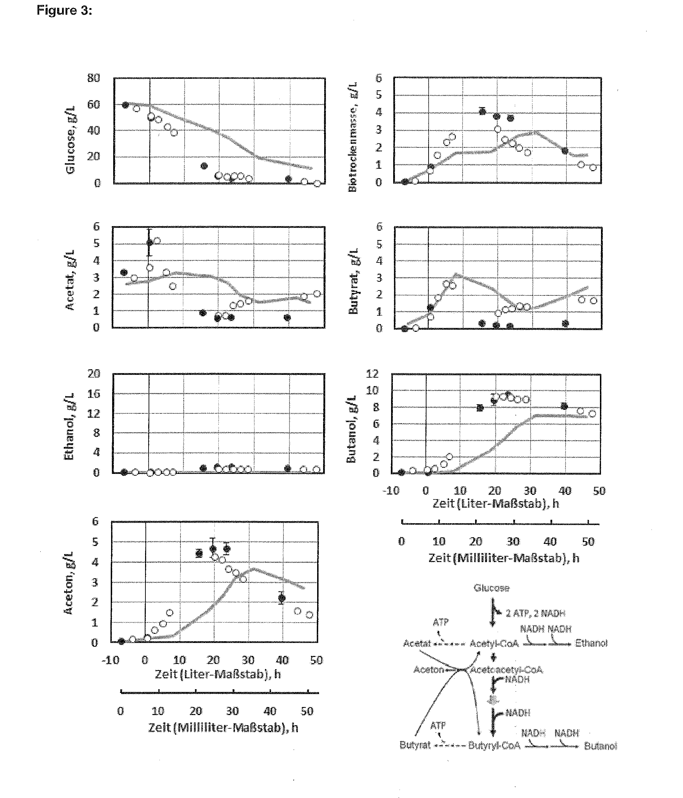

**Figure 3** shows product concentrations during the batch cultivation of *C*. *acetobutylicum* OverExpression-pT::crt and compares them to the wild type strains performance. **Figure** 3 **legend: Characterization of** *C. acetobutylicum* **crt overexpression strain:** Utilization of glucose, production of biomass, production of organic acids acetate, butyrate, and production of solvents acetone, ethanol and butanol is shown. White circles present performance of the crt overexpression strain in liter scale reactor and black circles present strain performance in milliliter scale reactor. Performace of maternal strain with *wt* genotype is presented with grey line. Error bars stay for standard deviation from 3 independent experiments.

### DETAILED DESCRIPTION OF THE INVENTION

The cells for use in the method of the present invention are Clostridium cells with the ability to produce butanol, wherein the cell comprises a genetic modification that results in overexpression of one or more of the following genes:
- a gene encoding crotonase (*crt*),
- a gene encoding butyryl-CoA dehydrogenase (*bcd*),
- a gene encoding 3-hydroxybutyryl-CoA dehydrogenase *(hbd).*

The genetically modified Clostridium cell is preferably a cell or strain from *C*. *acetobutylicum, C*. *beijerinckii, C*. *saccharoperbutylacetonicum* and *C*. *saccharobutylicum,* with *C*. *acetobutylicum* being preferred. Wild-type *C*. *acetobutylicum* is a strain that naturally produces the solvents acetone, butanol and ethanol in a ratio of 3:6:1. The sequence of the operon containing *crt, bcd* and *hbd* (as well as etfA and etfB) is given under http://www.ncbi.nlm.nih.gov/nuccore/U17110.1 (Accession is U17110.1). These genes are chromosomal. Suitable wild-type strains for genetic modification include *C*. *acetobutylicum* ATCC824, *C*. *acetobutylicum* DSM1731, *C*. *acetobutylicum* ATCC4529, and *C*. *acetobutylicum* M5.

The presence of a genetic modification that results in overexpression of one or more of the genes *crt, bcd,* and *hbd* can be verified by the presence of genetic elements that cannot be found in wild-type strains and that have a positive impact on the relevant enzymatic activity. Such genetic elements are particularly additional copies of one or more of *crt, bcd* and *hbd.* These additional copies have preferably a nucleic acid sequence encoding an amino acid sequence which is at least 70 % , more preferably at least 80 %, even more preferably at least 90 % such as at least 95 % or even 100 % identical to the amino acid sequence of SEQ ID No. 1, having crotonase activity, and/or a sequence encoding an amino acid sequence which is at least 70 %, more preferably at least 80 %, even more preferably at least 90 % such as at least 95 % or even 100 % identical to the amino acid sequence of SEQ ID No. 2, having butyryl-CoA dehydrogenase activity, and/or a sequence encoding an amino acid sequence which is at least 70 %, more preferably at least 80 %, even more preferably at least 90 % such as at least 95 % or even 100 % identical to the amino acid sequence of identical to the amino acid sequence of SEQ ID No. 3, having 3-hydroxybutyryl-CoA dehydrogenase.

Comparison of sequence identity (alignments) is performed using the ClustalW Algorithm (Larkin M.A., Blackshields G., Brown N.P., Chenna R., McGettigan P.A., McWilliam H., Valentin F., Wallace I.M., Wilm A., Lopez R., Thompson J.D., Gibson T.J. and Higgins D.G. (2007) ClustalW and ClustalX version 2. Bioinformatics 2007 23(21): 2947-2948).

These additional copies may be present in the cell's chromosome, in episomes or in plasmids. Additionally, or alternatively, such genetic elements may be stronger native promoters (lie the thl promoter) that replace the wild-type promoters of the chromosomally located *crt, bcd,* and *hbd* (which can be effected e.g. by homologous recombination). Furthermore, one may usually conclude from such an analysis of the genetic elements which (wild-type) strain was used for genetic modification. (For example, the entire wild-type *Clostridium acetobutylicum* ATCC824 genome is shown in http://www.ncbi.nlm.nih.gov/nucoore/NC 003030; Accession is NC_003030). If the wild-type strain is available for comparison, the genetic modification can also be verified by an increased enzymatic activity of one or more of Crt, Bcd, and Hbd in comparison to the wild-type strain. The increased enzymatic activity can e.g. be shown by an increased butanol production.

The genetic modification can be a genetic modification that results in overexpression of one of *crt, bcd* and *hbd,* a genetic modification that results in **overexpression** of two of *crt, bcd* and *hbd,* or a genetic modification that results in overexpression of all three of *crt, bcd* and *hbd.*

The overexpressed gene(s) is preferably homologous to the Clostridium cell. Alternatively, it may be heterologous to the Clostridium cell. For example, the genes may originate from any other species belonging to the class of Actinobacteria.

The genetic modification is preferably the result of a transformation with one or more vectors comprising one or more of the genes *crt, bcd* and *hbd.* The vectors preferably contain a nucleic acid sequence encoding an amino acid sequence which is at least 70 % , more preferably at least 80 %, even more preferably at least 90 % such as at least 95 % identical to the amino acid sequence of SEQ ID No. 1, and/or a sequence encoding an amino acid sequence which is at least 70 %, more preferably at least 80 %, even more preferably at least 90 % such as at least 95 % identical to the amino acid sequence of SEQ ID No. 2, and/or a sequence encoding an amino acid sequence which is at least 70 %, more preferably at least 80 %, even more preferably at least 90 % such as at least 95 % identical to the amino acid sequence of identical to the amino acid sequence of SEQ ID No. 3.

In the vector, the gene is operatively linked to a promoter. A preferred promoter is the promoter of C.acetobutylicum ATCC 824 thiolase gene having the sequence of SEQ ID No. 4., or a functional equivalent having a sequence identity of at least 90, more preferably 95 %.

In a particularly preferred embodiment, the vector is a shuttle vector for replication in *E.coli* and *C*. *acetobutylicum.*

Suitable promoters and vectors are described in Girbal et al., Applied and Environmental Microbiology 2005, Vol. 71, No. 5, p.2777-2781 and Mann et al. Biotechnol.Lett (2012) 34: 1643-1649.

The cells of the present invention can be produced by a method comprising the steps of
(i) providing a wild-type Clostridium strain;
(ii) amplifying one or more of the following genes of said strain:
   - a gene encoding crotonase *(crt),*
   - a gene encoding butyryl-CoA dehydrogenase (*bcd*),
   - a gene encoding 3-hydroxybutyryl-CoA dehydrogenase *(hbd),*
(iii) cloning the amplified DNA sequences into plasmids;
(iv) transforming the wild-type Clostridium strain with the plasmids obtained in step (iii);
(v) selecting strains that show a higher activity of the enzyme encoded by the amplified gene than the wild-type Clostridium strain.

It is particularly preferred that the plasmids are transformed into E.coli for methylation prior to step (iv).

The invention provides an improved process for producing butanol using Clostridia strains with increased metabolic fluxes through butyryl-CoA synthesis. The fluxes were increased in *Clostridium acetobutylicum strain* by homologous overexpression of butyryl-CoA synthesis genes. The present invention also provides a method for producing butanol, comprising the steps of fermenting a medium containing a sugar, or a precursor thereof, with a Clostridium cell of the present invention, wherein the Clostridium cell ferments sugar to butanol, and recovering butanol. The Clostridium cell also preferably ferments sugar to ethanol. Most preferably butanol and ethanol are generated in a molar ratio in the range of 1:1 to 3:1. More preferably, the maximum butanol concentration is at least 150 mM (e.g. in the range of 160 to 200 nM), and the maximum ethanol concentration is at least 60 mM (e.g. in the range of 100 to 150 nM). Acetate is preferably generated in a molar ratio butanol:acetate of at least 8:1, preferably at least 12:1, more preferably at least 20:1. The overall ABE titers are preferably in the range above 20 g/I ABE. These values can be determined e.g. after 20 hours of fermentation.

The process is preferably carried out in batch mode. The reaction volume comprising the sugar, or precursor thereof and the bacteria is preferably 1 I or more, more preferably 10 I or more. The reaction time is preferably in the range of 15 to 25 hours.

### SEQUENCES

SEQ ID: No: 1: crotonase 
MELNNVILEKEGKVAVVTINRPKALNALNSDTLKEMDYVIGEIE

NDSEVLAVILTGAGEKSFVAGADISEMKEMNTIEGRKFGILGNKVFRRLELLEKPVIA

AVNGFALGGGCEIAMSCDIRIASSNARFGQPEVGLGITPGFGGTQRLSRLVGMGMAKQ

LIFTAQNIKADEALRIGLVNKVVEPSELMNTAKEIANKIVSNAPVAVKLSKQAINRGM

QCDIDTALAFESEAFGECFSTEDQKDAMTAFIEKRKIEGFKNR
SEQ ID No. 2: putative butyryl-CoA dehydrogenase 
MDFNLTREQELVRQMVREFAENEVKPIAAEIDETERFPMENVKK

MGQYGMMGIPFSKEYGGAGGDVLSYIIAVEELSKVCGTTGVILSAHTSLCASLINEHG

TEEQKQKYLVPLAKGEKIGAYGLTEPNAGTDSGAQQTVAVLEGDHYVINGSKIFITNG

GVADTFVIFAMTDRTKGTKGISAFIIEKGFKGFSIGKVEQKLGIRASSTTELVFEDMI
 
VPVENMIGKEGKGFPIAMKTLDGGRIGIAAQALGIAEGAFNEARAYMKERKQFGRSLD

KFQGLAWMMADMDVAIESARYLVYKAAYLKQAGLPYTVDAARAKLHAANVAMDVTTKA

VQLFGGYGYTKDYPVERMMRDAKITEIYEGTSEVQKLVISGKIFR
SEQ ID No. 3: 3-hydroxybutyryl-CoA dehydrogenase 
MKKVCVIGAGTMGSGIAQAFAAKGFEVVLRDIKDEFVDRGLDFI

NKNLSKLVKKGKIEEATKVEILTRISGTVDLNMAADCDLVIEAAVERMDIKKQIFADL

DNICKPETILASNTSSLSITEVASATKTNDKVIGMHFFNPAPVMKLVEVIRGIATSQE

TFDAVKETSIAIGKDPVEVAEAPGFVVNRILIPMINEAVGILAEGIASVEDIDKAMKL

GANHPMGPLELGDFIGLDICLAIMDVLYSETGDSKYRPHTLLKKYVRAGWLGRKSGKG

FYDYSK
SEQ ID: No : 4 : Thl prmoter 135 bp:

tcgactttttaacaaaatatattgataaaaataataatagtgggtataattaagttgttagagaaaacgtataaattagggataaactatgga

acttatgaaatagattgaaatggtttatctgttaccccgtag

### EXAMPLES

### Example 1

### Cloning and homologous overexpression of BCS genes

In order to improve the metabolic fluxes through the butyryl-CoA synthesis (BCS) pathway, the BCS genes of *Clostridium acetobutylicum* were homologously overexpressed for increased dosages of 3-hydroxybutyryl-CoA dehydrogenase (Hbd), crotonase (Crt) and butyryl-CoA dehydrogenase (Bcd) complex.. To accomplish this, the genes *hbd* (CAC2708), *crt* (CAC2712) and *bcd*/*etfAB* (CAC2709-2711) were amplified by PCR from chromosomal DNA of *C. acetobutylicum* ATCC 824 (Fischer et al. , J. Bacteriol, August 2006; 5469-5478) using the oligonucleotides listed in TABLE 1 as PCR primers. The DNA fragments were cloned via BamHI and *Kas*I restriction sites into the pT*HydA* plasmid as described (Girbal et al. 2005; Hillmann F, Döring C, Riebe O, Ehrenreich A, Fischer RJ and Bahl H, 2009. The role of PerR in O2-affected gene expression of Clostridium acetobutylicum. J. Bacteriol. 191:6082-6093.; Mann et al. 2012).

**TABLE 1: Oligonucleotides used for gene cloning. Genes were amplified from chromosomal DNA of C. acetobutylcium ATCC 824 and purified PCR products were cloned into the pT vector via BamHI and KasI restriction sites (underlined).**

| **Gene (ORF)** | **Primer** | **Sequence (5' → 3')** |
|---|---|---|
| *hbd* (CAC2708) | hbd_fw_BamHI | AAAAAGGATCCATGAAAAAGGTATGTG |
| | hbd_rv_KasI | AAAAAGGCGCCTTATTTTGAATAATCGTAG |
| *crt* (CAC2712) | crt_fw_BamHI | AAAAAGGATCCATGGAACTAAACAATG |
| | crt_rv_KasI | AAAAAGGCGCCCTATCTATTTTTGAAGCC |
| *bcd*/*etfAB* (CAC2709-2711) | bcd_etfAB_fw_BamHI | AAAAAGGATCCATGGATTTTAATTTAACAAG |
| | bcd_etfAB_rv_KasI | AAAAAGGCGCCTTAATTATTAGCAGCTTTAAC |

The resulting plasmids pT::*hbd*, pT::*crt* and pT::bcd, respectively, were transformed into *E. coli* DH5a and validated by DNA sequencing (LGC Genomics GmbH, Berlin, Germany). *E. coli* strains were cultivated in LB medium comprising per liter 5 g yeast extract, 10 g tryptone and 10 g NaCl, ampicillin was added for plasmid maintenance at a concentration of 100 µg/ml (Sambrock J and Russell DW, 2001. Molecular Cloning: A Laboratory Manual, 3rd Ed. Cold Spring Harbor Laboratory Press, NY, USA.). After *in vivo* methylation in *E. coli* ER2275 pAN2, the plasmids were transformed into *C*. *acetobutylicum* ATCC 824 by electroporation and respective overexpression strains were selected on reinforced clostridial agar (RCA, Oxoid Deutschland GmbH, Wesel, Germany) containing 40 µg ml⁻¹ erythromycin.

### Example 2

### Effect of BCS gene overexpression in C. acetobutylicum

The recombinant strains of *C*. *acetobutylicum* were subjected to fermentation experiments at a 200 ml-scale in a defined synthetic medium to assess the phenotypic characteristics. The parental wildtype *C*. *acetobutylicum* ATCC 824 and the vector control strain *C, acetobutylicum* ATCC 824/pT (Mann et al. 2012) were used as references.

In general, recombinant *C. acetobutylicum* strains were cultivated anaerobically at 37°C without shaking in Hungate tubes or serum bottles, 40 µg/ml erythromycin were added for plasmid maintenance. Resazurin (7-hydroxy-10-oxidophenoxazin-10-ium-3-one) was added as a redox indicator for anaerobiosis at a concentration of 1 mg/l and residual oxygen was removed by addition of 50-100 µl titanium (III) nitrilotriacetic acid (NTA) solution (1.3 M NaOH, 0.16 M NTA, 0.27 M Na₂CO₃ and 1.3 % TiCl₃).

Fermentation experiments were performed in 200 ml MS-MES medium in serum bottles (Müller & Krempel AG, Bülach, Switzerland) with the following composition per liter: 0.55 g KH₂PO₄, 0.55 g K₂HPO₄, 0.22 g MgSO₄ x 7 H₂O, 0.011 g FeSO₄ x 7 H₂O and 2.3 ml acetic acid; after the pH was adjusted to 6.6 with NH₄OH, 40 µg p-aminobenzoic acid, 0.32 µg biotin, 1 mg resazurin and 21.3 g 2-(N-morpholino) ethanesulfonic acid (MES) were added (modified from Monot, F et al, 1982, Appl. Environ. Microbiol. 44, 1318-1324). glucose was used as carbon source at a concentrations of 60 g/l. Samples were regularly drawn to monitor growth and cell-free supernatant samples were stored at -20°C until quantification of glucose and fermentation products. Acetate, butyrate, acetone, ethanol and butanol were determined as described previously on an Agilent 7890A gas chromatograph (Agilent Technologies, Böblingen, Germany) equipped with a Chromosorb 101 (80/100 mesh, 2.0 m x 3.0 mm x 1.6 mm) glass column.

**TABLE 2: Fermentation products of recombinant C. acetobutylicum-strains. Mean values and standard deviations after 88 h of cultivation of three independent replicates per strain are shown.**

| **Products** | ***C. acetobutylicum* strain** | | | | |
|---|---|---|---|---|---|
| | **Wildtype** | **pT** | **pT::hbd** | **pT::*crt*** | **pT::*bcd*** |
| Acetate (mM) | 5.9 ± 1.8 | 15.6 ± 5.2 | 8.6 ± 1.2 | 14.2 ± 0.6 | 18.1 ±5.8 |
| Butyrate (mM) | 0 | 0 | 0 | 0 | 0 |
| Acetone (mM) | 63.8 ± 4.1 | 82.0 ± 3.1 | 114.5 ± 11.9 | 83.1 ± 2.3 | 81.1 ± 1.1 |
| Ethanol (mM) | 32.1 ± 2.1 | 21.7 ± 5.6 | 124.1 ± 1.0 | 140.8 ± 3.9 | 62.0 ± 2.4 |
| Butanol (mM) | 139.4 ± 3.4 | 139.1 ± 2.3 | 180.6 ± 1.9 | 172.7 ± 2.4 | 166.4 ± 4.4 |

**F*i*gure 1** shows product concentrations of (Acetone-Butanol-Ethanol = ABE) and yields on glucose of recombinant *C. acetobutylicum*-strains.

Interestingly, all three novel overexpression strains exhibited a significantly improved solvent production, i. e. acetone, butanol and ethanol (ABE). The overall ABE titers were increased to 25.7 ± 0.9 g/l (*hbd* overexpression), 24.1 ± 0.5 g/I (*crt* overexpression) and 19.9 ± 0.5 g/I (*bcd*/*etfAB* overexpression), respectively, as compared to the vector control strain with 16.1 ± 0.6 g/l ABE (FIGURE 1). Whereas n-butanol was the main fermentation product in all recombinant *C. acetobutylicum* strains, *hbd* and *crt* overexpression also resulted in higher ethanol formation (TABLE 2). These results clearly indicate that increasing BCS pathway enzymes by gene overexpression constitutes a suitable metabolic engineering strategy to overcome natural limits in clostridial butanol production. Such approach was for first time demonstrated in this publication.

### Example 3

### Enzyme activities in recombinant C. acetobutylicum strains

To validate increased BCS enzyme dosages, the Hbd and Crt as well as the thiolase activities were measured in the recombinant *C. acetobutylicum* strains and compared to the wildtype and vector control strains.

For the determination of enzyme activities in crude extracts, cells of *C. actetobutylicum* grown in MS-MES were harvested in the late expontential growth, and cells were stored at -70°C when necessary. All following steps were done at 4°C or on ice, respectively. The cell pellets were resuspended in 100 mM Tris/HCl buffer, pH 7.6, and disrupted by sonication using a Hielscher UP200S ultrasonic processor equipped with the sonotrode S3 (Hielscher Ultrasonics GmbH, Teltow, Germany). The cell suspension was sonicated for 90 s at 80 % amplitude, followed by a break for 60 s, this cycle was repeated 8-12 times. When necessary, breaks of 4-5 min were included to prevent warming of the suspension, the process was controlled by light microscopy. The cell extract was centrifugated at 13,000 rpm for 30 min and the supernatant was used for enzyme assays as described below. The protein content was determined according to Bradford (1976).

Thiolase activity was measured spectrophotometrically by acetoacetyl-CoA decrease at 303 nm. 3-Hydroxybutyryl-CoA dehydrogenase (Hbd) activity was determined by NADH decrease at 340 nm according to Madan et al. (1973). Crotonase activity was measured by crotonyl-CoA decrease at 263 nm as described previously.

As summarized in TABLE 3, Hbd and Crt activities were significantly increased in *C. acetobutylicum* pT::*hbd* and *C. acetobutylicum pT::crt,* respectively. Interestingly, activities of the thiolase, the first enzyme of the BCS pathway catalyzing the condensation of two molecules acetyl-CoA, was not significantly changed in any overexpression strain as compared to the wildtype and the vector control strain.

**TABLE 3: Enzyme activities of recombinant C. acetobutylicum-strains. Average values and standard deviations of three independent measurements are shown. One unit of activity corresponds to the conversion of one millimole substrate per minute per milligram protein content of the crude extracts.**

| ***C.** acetobutylicum* **strain** | **Thiolase (U/mg)** | **3-Hydroxybutyryl-CoA dehydrogenase (U/mg)** | **Crotonase (U/mg)** |
|---|---|---|---|
| Wildtype | 31.5 ± 1.2 | 10.3 ± 0.5 | 94.9 ± 4.5 |
| pT | 29.9 ± 0.6 | 10.8 ± 0.6 | 97.8 ± 7.1 |
| pT::*hbd* | 29.7 ± 1.3 | 19.0 ± 0.8 | 100.9 ± 2.5 |
| pT::*crt* | 31.9 ± 0.4 | 11.5 ± 0.3 | 160.2±4.4 |
| pT::*bcd* | 34.2 ± 2.0 | 9.9 ± 0.8 | 91.4 ± 2.2 |

### Example 4

### Clostridium preculture preparation

*C. acetobutylicum*ATCC 824, *Clostridium acetobutylicum* ÜE-pT::*bcd*, *Clostridium acetobutylicum* ÜE-pT::*crt*), were cultivated anaerobically at 37°C. Precultures were obtained from spore suspensions inoculated to clostridial growth medium in anaerobic flasks with a liquid volume of 5 mL (glucose, 2.5 g L⁻¹; KH₂PO₄, 0.75 g L⁻¹; K₂HPO₄, 0.75 g L⁻¹; MgSO₄·7 H₂O, 0.4 g L⁻¹; MnSO₄ ·H_{z}O, 0.01 g L⁻¹; FeSO₄ ·7 H₂O, 0.01 g L⁻¹; NaCl, 1 g L⁻¹; (NH₄)₂SO₄, 2 g L⁻¹; yeast extract, 5 g L⁻¹; asparagine, 2 g L⁻¹; pH 6.6 adjusted with NH₄OH) and pasteurized at 80°C for 10 min. After an initial growth phase of 16 h, the germed culture suspension was transferred to a modified mineral salt 2-(N-morpholino) ethanesulfonic acid medium (MS-MES medium; 10% v/v). MS-MES medium was prepared in anaerobic flasks (45 mL working volume) with glucose (60 g L⁻¹), KH₂PO₄ (0.55 g L⁻¹), K₂HPO₄ (0.42 g L⁻¹), MgSO₄ · 7 H₂O (0.22 g L⁻¹), FeSO₄ · 7 H₂O (0.011 g L⁻¹), 10.08 mg L⁻¹ biotin and 8 mg L⁻¹ p-aminobenzoic acid, (NH₄)₂SO₄ (5.496 g L⁻¹) and acetic acid (2.3 g L⁻¹) at pH 5.5 adjusted with KOH (modified from Monot, F et al, 1982, Appl. Environ. Microbiol. 44, 1318-1324). 18 mg L⁻¹ ZnSO₄ · 7 H₂O were added, if declared.The exponentially growing microorganisms were transferred after 16 h into fresh MS-MES medium in anaerobic flasks. This procedure was repeated until sufficient cells were available for the inoculation of a stirred-tank reactor. Before inoculation the optical density (OD) was measured at 600 nm to adjust the inoculation volume to achieve an initial dry cell weight of 0.15 g L⁻¹ in the stirred-tank bioreactors filled with sterile MS-MES medium.

### Example 5

### Cultivations on a milliliter scale

The parallel bioreactor system was applied with baffled single-use bioreactorsmade of polystyrene. To ensure initial anaerobic conditions, the system and all necessary componentswere stored in an anaerobic chamber overnight before each of the parallel bioreactorswith a nominal volume of 20 mL was filled with 12 mL of an inoculated MS MES medium with0.1 mL L⁻¹ polypropylene glycol as an antifoaming agent. The inoculated parallel bioreactor system was manually transferred to the control station outside the glove box. Immediately afterwards it wasgassed with 48 L h⁻¹ of N₂ for 1 h to ensure anaerobic conditions. The sterile gas cover of the parallelbioreactor system ensured identical gas flows into the headspaces of each parallel stirred-tank bioreactor. Afterwards the total gassing rate was reduced to 6 L h⁻¹ (≈ 2.1 mL min⁻¹ for each parallel bioreactor), the cultivation temperature was adjusted to 37°C, and headspace cooling was set to 2°C to reduce evaporation. This headspace cooling temperature resulted in a volume-specific evaporationrate identical to that on the liter scale (data not shown). For this purpose, evaporation experiments without microorganisms were performed under these conditions. The volume-specific evaporation rates of butanol, acetone, and ethanol were 0.06, 0.14, and 0.01 g L⁻¹ h⁻¹, respectively, on both scales. Evaporation of water was negligible. Because energy dissipation is a relevant scale criteria for the cultivation of *C. acetobutylicum* the stirrer speed at the milliliter scale was adjusted to 400 rpm, which resulted in a power supply of 0.2 W L⁻¹ . Due to this relatively low rotationalspeed, no gas phase was sucked in by the gas-inducing stirrers for dispersion of gas bubbles into the fermentation medium.

### Example 6

### Cultivations on a liter scale

The reference batch process was carried out at 37°C in a 2 L stirred tank reactor (Labfors 3, Infors, Switzerland) with 2 Rushton turbine impellers at a working volume of 1 L. The pH was monitored with
the control software Iris NT Pro v 5.02 (Infors-HT, Bottmingen, Switzerland). After heat sterilization (121°C, 20 min) of the reactor with MS-MES medium, the reactor was immediately sparged with sterile nitrogen gas (2 L min⁻¹, 5.0 Nitrogen; Air Liquide, Munich, Germany) while being cooled to establish and remain anaerobic conditions. At a temperature of 37°C gassing was reduced to 0.17 L N₂ min⁻¹. After inoculation, the initial pH was manually adjusted to pH 5.5 with KOH. The stirrer speed was increased from 50 rpm to 200 rpm after the pH dropped down to pH 5.4 (power consumption at 200 rpm for the liter-scale system: 0.2 W L⁻¹; . The fermentation process time was initiated (*t* = 0 h) when the stirrer speed was increased to 200 rpm, because the initial lag phases varied extremely. The evaporation of water was negligible due to the low gassing rate.

**Figure 2** shows changes in product concentrations during the batch cultivation in stirred tank reactors of *C. acetobutylicum* OverExpression--pT::bcd are shown and compared to the wild type strains performance.

The process time axis of the cultivations on a mL scale was delayed for 10 hours. With this delay both scales show the same process performance for the recombinant strain.
The courses of all concentrations of the recombinant strain on both scales were comparable. Glucose was consumed completely at a process time of 20 hours. This resulted in a glucose consumption rate of 2.4 g/(Lh). The wild type strain showed a glucose consumption rate of 0.9 g/(Lh) at a process time of 20 hours and at a process time of 50 hours a rest glucose concentration of 10 g/L remained in the medium. The maximum cell dry weight of 4 g/L of *C. acetobutylicum* ÜE-pT::bcd was 35 % higher and was obtained 10 h earlier compared to the wild type strain. The maximum acid concentrations were not determined, but the qualitative courses are lower compared to that of the wild types. This may be a proof for a better reassimilation of acids. Ethanol production was a bit higher than the wild type strains.

An earlier onset of butanol production and a significant increase in butanol production were evident. This resulted in higher butanol concentrations. At a process time of 14 hours butanol concentrations of 6 g/L were produced by the recombinant strain while the wild type strain produced only 2 g/L. At a process time of 20 hours a butanol concentration of 9 g/L were produced by the recombinant strain, while the wild type strain produced a maximal butanol concentration of 7 g/L after 31 hours.

The course of the acetone concentration was qualitatively comparable. The onset of the production of the recombinant strain occurred earlier and a maximum concentration of 4.4 g/L was reached at 20 h. The wild type strain reached a maximum acetone concentration of 4 g/L after 31 h. The decrease in cell dry mass immediately after reaching the maximum butanol concentration is noticeable with both strains.

The data clearly demonstrate that bcd overexpression led to faster kinetics butanol production and to higher butanol yields. This is a first demonstration of butanol production improvement achieved by gene overexpression on reactor scale. Such improvement exceeds expectations and it is relevant for production level.

**Figure 3** shows product concentrations during the batch cultivation of *C*. *acetobutylicum* OverExpression-pT::crt and compares them to the wild type strains performance.

In contrast to the recombinant OverExpression-pT::bcd strain, the *C. acetobutylicum* OverExpression -pT::crt strain did not consume all glucose at a process time of 20 hours. 6 g/L glucose concentration remained in the medium at this process time but this is considerably less compared to the performance of the wild type strain (35 g/L glucose concentration at t = 20 h). The glucose consumption rates of *C. acetobutylicum* OverExpression-pT::crt were 250 % increased (2,3 g/L compared to 0,9 g/L wild type glucose consumtion rate). The maximum cell dry weight of 4 g/L of *C. acetobutylicum* OverExpression--pT::crt was 35 % higher and was obtained 10 h earlier compared to the wild type strain.

The butyrate concentrations were comparable to the one of the wild type strain but the acetate concentration was increased to 5 g/L compared to 3 g/L reached with the wild type strain before the onset of reassimilation.

The solventogenic phase proceeded comparably to *C*. *acetobutylicum* OverExpression-pT::bcd. At a process time of 20 hours the maximal solvent concentrations were obtained (9 g/L butanol concentration and 1 g/L ethanol concentration and 4,3 g/L acetone).

Again the data clearly demonstrate that bcd overexpression led to faster kinetics of butanol production and to higher butanol yields. This is a first demonstration of butanol production improvement achieved by gene overexpression on reactor scale. Such improvement exceeds expectations and it is relevant for production level.

## Claims

1. A Clostridium cell with the ability to produce butanol, wherein the cell comprises a genetic modification that results in overexpression of one or more of the following genes:
• a gene encoding crotonase (*crt*),
• a gene encoding butyryl-CoA dehydrogenase *(bcd),*
• a gene encoding 3-hydroxybutyryl-CoA dehydrogenase (*hbd*).

2. The Clostridium cell of claim 1, wherein the genetic modification is the result of a transformation with a vector comprising one or more of the following genes:
• a gene encoding crotonase (*crt*),
• a gene encoding butyryl-CoA dehydrogenase (*bcd*),
• a gene encoding 3-hydroxybutyryl-CoA dehydrogenase (*hbd*).

3. The Clostridium cell of claim 1 or 2, wherein the gene(s) are homologous to the Clostridium cell.

4. The Clostridium cell of claim 1 or 2, wherein the gene(s) are heterologous to the Clostridium cell.

5. The Clostridium cell of claim 4, wherein the gene(s) is overexpressed by transforming the cell with a crotonase gene or a butyryl-CoA dehydrogenase gene originating from species belonging to class of Actinobacteria.

6. The Clostridium cell of one or more of the preceding claims, wherein the Clostridium cell belongs to the species *Clostridium acetobutylicum.*

7. The Clostridium cell of claim 1, wherein the Clostridium cell is derived from *Clostridium acetobutylicum* ATCC 824.

8. A vector for transforming a Clostridium cell, comprising one or more of the following genes:
• a gene encoding crotonase (*crt*),
• a gene encoding butyryl-CoA dehydrogenase (*bcd*)*,*
• a gene encoding 3-hydroxybutyryl-CoA dehydrogenase (*hbd*).

9. The vector of claim 8, comprising one or more of the following sequences:
• a sequence encoding an amino acid sequence which is at least 70 % identical to SEQ ID No. 1,
• a sequence encoding an amino acid sequence which is at least 70 % identical to SEQ ID No. 2,
• a sequence encoding an amino acid sequence which is at least 70 % identical to SEQ ID No. 3.

10. A method for producing the cell according to one or more of claims 1 to 7, comprising the steps of
(vi) providing a wild-type Clostridium strain;
(vii) amplifying one or more of the following genes of said strain:
• a gene encoding crotonase (*crt*),
• a gene encoding butyryl-CoA dehydrogenase (*bcd*),
• a gene encoding 3-hydroxybutyryl-CoA dehydrogenase (*hbd*),
(viii) cloning the amplified DNA sequences into plasmids;
(ix) transforming the wild-type Clostridium strain with the plasmids obtained in step (iii);
(x) selecting strains that show a higher activity of the enzyme encoded by the amplified gene than the wild-type Clostridium strain.

11. The method of claim 10, wherein the plasmids are transformed into E.coli for methylation prior to step (iv).

12. A method for producing butanol, comprising the steps of fermenting a medium containing a sugar, or a precursor thereof, with a Clostridium cell of one or more of the claims 1 to 7, wherein the Clostridium cell ferments sugar to butanol, and recovering butanol.

13. The method of claim 12, wherein the Clostridium cell also ferments sugar to ethanol.

14. The method of claim 13, wherein butanol and ethanol are generated in a molar ratio in the range of 1:1 to 3:1.

15. The method of claim 13 or 14, wherein acetate is generated in a molar ratio butanol:acetate of at least 8:1.
